# EUROPÄISCHE PATENTSCHRIFT

(11) **EP 1 641 404 B1**
(45) Veröffentlichungstag und Bekanntmachung des Hinweises auf die Patenterteilung: **16.03.2011**
(21) Anmeldenummer: 03817328.2
(22) Anmeldetag: 03.07.2003
(51) Int. Cl.: A61B 17/80

(54) **OSTEOSYNTHESESPIRALE / OSTEOSYNTHESESPIRALENSYSTEM**
OSTEOSYNTHESIS SPIRAL / OSTEOSYNTHESIS SPIRAL SYSTEM
SPIRALE D'OSTEOSYNTHESE / SYSTEME DE SPIRALE D'OSTEOSYNTHESE

(43) Veröffentlichungstag der Anmeldung: 05.04.2006
(73) Patentinhaber: Ebid, Rainer, 82024 Taufkirchen (DE)
(72) Erfinder: Ebid, Rainer, 82024 Taufkirchen (DE)
(86) Internationale Anmeldenummer: PCT/EP2003/007086
(87) Internationale Veröffentlichungsnummer: WO 2005/002455

(56) Entgegenhaltungen:
- WO-A-00/71031
- WO-A-03/007831
- US-A- 4 403 606
- US-A- 5 916 200
- US-A- 5 984 926

## Beschreibung

### Stand der Technik

Zur Osteosynthese werden derzeit verschiedene Systeme verwendet. Neben der kaum noch gebräuchlichen Drahtnahtosteosynthese gibt es Platten, Schrauben, Krampen und Gitter und Mesh (Gittergeflecht) in verschiedenen Ausführungen sowie verschiedene Systeme des Fixateur externe.

In "US 5 984 926 A" wird ein aus spiralig angeordneten flexiblen Fasern entstandene Netzwerk, welches zur Verankerung einer Schraube in einem Schraubenloch dient, beschrieben. Dieses Netzwerk wird dabei, wie beschrieben, wie ein "Strumpf" oder "Ärmel" über die Schraube gezogen und ist damit einem Dübel zur Verankerung von Schrauben in der Wand vergleichbar und kommt somit im Knochen zu liegen, während die Osteosynthesespirale - vergleichbar einer Osteosyntheseplatte - auf der Knochenoberfläche befestigt wird.

In "US 2002/0147453" (Basis für den Oberbegriff des Anspruchs 1) wird ein Apparat beschrieben, bestehend aus einem Applikator und Osteosyntheseplatten, wobei speziell auf die in "US 00000 4923471 A" beschriebenen Osteosyntheseplatten Bezug genommen wird, welche bekannte Standardformen von Osteosyntheseplatten darstellen. Der Applikator kann in Kombination mit jeder Platte verwendet werden. Dargestellt wird in "US 2002/0147453" eine Platte, welche aus einem zentralen Körper und - davon ausstrahlend - gebogenen Armen besteht.
In "US 5 916 200 A" strahlen verformbare gerade oder bogenförmige Finger von einem ringförmigen Körper, welcher in einer Variante nicht geschlossen ist, aus und dienen der Fixation des zu fixierenden Gegenstandes. In einer Variante werden konzentrische Ringe mittels Stegen miteinander verbunden.
Eine Spirale hat gegenüber den beschriebenen Körpern mit ausstrahlenden Fingern - entsprechend "US 5 916 200 A" - oder Armen, entsprechend "US 2002/0147453", mehr Möglichkeiten zur Wahl des Ortes, an welchem die Fixation mittels einer Schraube auf der Unterlage erfolgt, was besonders bei Trümmerfrakturen wichtig ist.

Die dreidimensionale Verformbarkeit einer Spirale unterschiedet sich grundsätzlich von einem Ring mit ausstrahlenden Fortsätzen, was bei der Adaptation an den Knochen wichtig ist.

Der in "US 5 916 200 A" dargestellte Gegenstand, bestehend aus konzentrischen Ringen und Stegen - welche die Ringe verbinden -, zerfällt bei Entfernung der Stege in mehrere Fragmente, während bei einer Spirale durch die Entfernung von Stegen zwischen zwei benachbarten Windungen eine erhöhte Verformbarkeit der Spirale erreicht wird, ohne den Zerfall der Spirale.

Abbildungen:
- Fig. 1: Beispiel für eine Osteosynthesespirale; Grundform vollständig rund
- Fig. 2: Beispiel für eine Osteosynthesespirale; Grundform dreieckig
- Fig. 3: Beispiel für zwei, an einem Ende miteinander verbundene Osteosynthesespiralen; Grundform vollständig rund
- Fig. 4: Beispiel für eine Osteosynthesespirale; Grundform vollständig rund
- Fig. 5: Beispiel für einen geraden Steg
- Fig. 6: Beispiel für einen Steg mit Schraubenloch
- Fig. 7: Beispiel für eine Schraube; a = Abstand zwischen innerem und äußerem Schraubendurchmesser.

### Erfindung

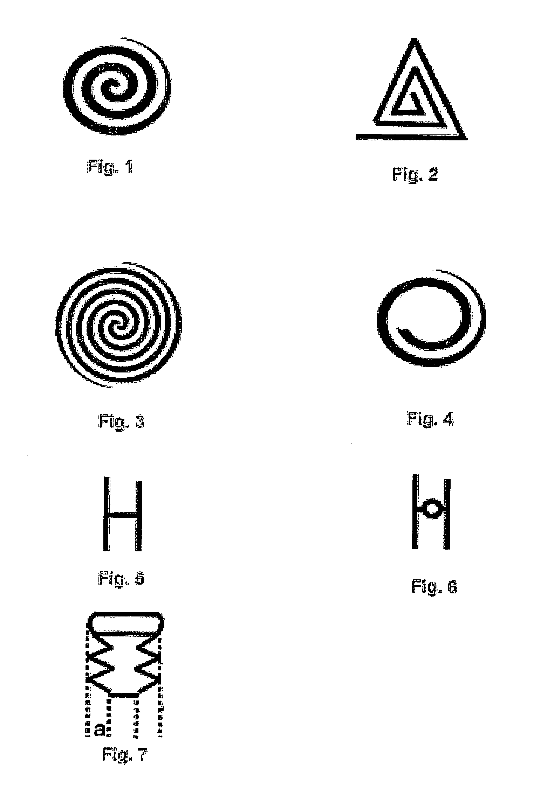

Die Osteosynthesespirale/Osteosynthesespiralensystem dient der Fixation von Knochen, Knochenfragmenten und künstlichen Körperteilen sowie Apparaturen zur Kallusdistraktion, mit Anwendungsbereich in der Humanmedizin und Tiermedizin. Die Grundform der Osteosynthesespirale/ Osteosynthesespiralensystem ist die Spirale. Die Form läßt sich folgendermaßen beschreiben. Ein Metallstab wird, ausgehend von einem zentralen Startpunkt, in streng parallelen Windungen nach peripher verlaufend gebogen. (Veranschaulicht ist dies in Fig. 1.)
In weiteren Ausführungen der Spirale sind Abschnitte der Windungen nicht gekrümmt sondern gerade, so dass von der runden Grundform der Spirale abweichende Formen entstehen, die je nach Ausführung auch zumindest eine Ecke beinhalten. Beispiele sind eine Hufeisenform, eine T-Form oder eine Dreiecksform. (Die Dreiecksform ist in Fig. 2 veranschaulicht.)
In weiteren Ausführungen sind zumindest zwei derartige Spiralen an zumindest einem Ende miteinander verbunden, so dass ein Spiralensystem entsteht. (Ein Beispiel ist in Fig. 3 veranschaulicht.)

In weiteren Ausführungen sind in die Spirale ein oder mehrere Schraubenlöcher integriert. Das erfordert je nach Ausführung eine breitere Dimensionierung der Windung an der Stelle des Schraubenloches. Je nach Ausführung ist ein Schraubenloch dabei zumindest Abschnittsweise durch eine runde oder gerade Linie oder eine Ecke begrenzt.

Je nach Ausführungsform sind die Enden der Spirale glatt auslaufend, zu einer Öse gebogen oder über einen Steg mit einer Nachbarwindung verbunden oder als Schraubenloch (siehe oben) ausgeformt.

Ein Quersteg verbindet zwei Punkte auf der Spirale anders als über die Windungen der Spirale. Entsprechend unterschiedlicher Ausführungen gibt es außer Spiralen ohne Quersteg Spiralen mit zumindest einem Quersteg. Die Ausführung des Quersteges richtet sich nach den anatomischen und physikalischen Anforderungen für das Anwendungsgebiet. (Ein Beispiel für einen Quersteg ist in Fig. 5 veranschaulicht.) Entsprechend unterschiedlichen Ausführungen bestehen außer Querstegen ohne Schraubenloch auch Querstege mit zumindest einem Schraubenloch (Ausführung siehe oben). (Ein Beispiel ist in Fig. 6 veranschaulicht.)

Entsprechend unterschiedlichen Ausführungen ist der Querschnitt des (oben erwähnten) Metallstabes, aus dem die Windungen geformt sind, in allen Bereichen gleich oder unterschiedlich. Die Begrenzung des Querschnittes ist, je nach Ausführungsform, rund oder eckig, abschnittsweise aus diesen Formen kombiniert oder eine der Varianten mit zumindest einem geraden Abschnitt kombiniert.

Als Sonderformen gelten Spiralen mit einfachen Windungen, mit Fixation jeweils beider Enden an der Nachbarwindung. Dabei entsteht ein Konstrukt aus 2 bzw. 3 parallel angeordneten, an den Enden miteinander verbundenen (=geschlossenen) Stäben. Ein einfach geschlossenes U ist ein in U-Form gebogener Stab mit paralleler Nachbarwindung, bei welchem beide Enden miteinander verbunden und somit an der Nachbarwindung fixiert sind.

Weitere Sonderformen sind Spiralen mit integrierten Löchern (z.B. für Schrauben oder anderes Fixationsmaterial), die an Osteosyntheseplatten erinnern. Das erfordert je nach Ausführung eine breitere Dimensionierung der Windung an der Stelle des Schraubenloches.

Je nach Ausführungsform ist die Osteosynthesespirale eigenständig oder ein integrativer Bestandteil in Kombination mit zumindest einer Osteosyntheseplatte, zumindest einem Osteosynthesegitter oder Mesh oder der Kombination aller drei Bestandteile oder integrativer Bestandteil eines Apparates zur Distraktionsosteogenese, einer Methode der Knochenneubildung.

Das Material ist - je nach Anwendungsbereich und damit notwendiger Stabilität - resorbierbar oder nicht resorbierbar. Derzeit bieten sich als nicht resorbierbare Materialien vor allem titan-haltige und andere biokompatible Metalle an.

Die Fixation am Knochen oder einem künstlichen Körperteil erfolgt mit Schrauben oder in einer anderen zur Fixation geeigneten Art und Weise. Die Schraubenfixation kann beispielsweise nahezu stufenlos zwischen 2 Windungen erfolgen. Dabei werden 2 Nachbarwindungen mittels Schraube an der Unterlage fixiert. Gleichzeitig wird die Schraube zwischen 2 Nachbarwindungen in die Unterlage eingedreht. Ein weiteres Beispiel ist die Fixation mittels Schraube an Quersteg und Windung oder innerhalb einer Windung (z.B. in der Ecke eines Dreiecks). Die Fixation im Schraubenloch, als weiteres Beispiel, erklärt sich von selbst.
Die Fixation kann eine Beilagscheibe - unter der Schraube - versehen mit einem zentralen Schraubenloch und 3 Domen, welche ein gleichseitiges Dreieck aufspannen, erfordern.
Die Beilagscheibe kann ansonsten jede beliebige Form haben und aus jedem beliebigen (resorbierbarem und nicht resorbierbarem) Material sein.

Eine weitere Möglichkeit besteht darin, den Abstand zwischen dem Außen- und Innenbereich der Schraubenwindungen so groß zu wählen, daß die Osteosynthesespirale eingeklemmt wird. (Veranschaulicht wird das in Fig. 7, Buchstabe a).

Die Osteosynthesespirale eignet sich besonders gut zur Osteosynthese im Bereich von Trümmerfrakturen, da eine fast stufenlose Wahl des Bohrloches für die Schraube möglich ist.

Zudem lässt sich die Osteosynthesespirale 3-dimensional modeltieren und eignet sich somit zur Rekonstruktion bei Defektfrakturen, z.B. Rekonstruktion bei konservativ nicht zufriedenstellend behandelbaren Frakturen (z.B. Nasenbeintrümmerfrakturen).

Bei Abdeckung eines großen Knochenareals besteht nur eine geringe Auflagefläche, was bei breiter flächiger Verteilung der Kraft auf der Unterlage die Ernährung des Knochens wenig stört.

Ohne Kontinuitätsunterbrechung der Spirale können zur Anpassung innere und äußere Windungen sowie, soweit vorhanden, Stege entfernt werden. Dies kann, was günstig ist, noch während der Operation geschehen.

## Patentansprüche

1. Osteosyntheseplatte welcher mittels Schrauben, vorzugsweise mittels Schrauben und Beilagscheibe, durch das Anpressen an der Knochenoberfläche befestigbar ist, wobei die Osteosyntheseplatte in Form einer Spirale aufgebaut ist,
**dadurch gekennzeichnet, dass**
die Spirale von einem zentralen Punkt startet und sich in gebogene oder echigen Windungen, welche jeweils parallel zueinander nach peripher verlaufen, erstreckt,
wobei der durch die Schraube fixierte Abschnitt der Windung der Spirale durch den Schraubenkopf oder die Beilagscheibe auf die Knochenoberfläche gepresst und dort gehalten wird.

2. Osteosyntheseplatte gemäß Anspruch 1 **dadurch gekennzeichnet, daß** die Windungen gebogene und eckige Abschnitte enthalten. 2

3. Osteosyntheseplatte gemäß einem der Ansprüche 1 bis 2, **dadurch gekennzeichnet, daß** einzelne Abschnitte der Spirale nicht gebogen sondern gerade sind insbesondere die Spirale eine Hufeisenform, eine T -Form oder die Form eines Dreiecks aufweist.

4. Osteosyntheseplatte gemäß einem der Ansprüche 1 bis 3, **dadurch gekennzeichnet, dass** die Spirale zumindest ein Verankerungselement in Form eines Schraubenloches enthält, wobei die Begrenzung des Schraubenloches vorzugsweise rund, eckig oder eine Kombination daraus, oder eine der drei Varianten mit kombiniert geraden Abschnitten ist.

5. Osteosyntheseplatte gemäß Anspruch 4, **dadurch gekennzeichnet, daß** die Enden der Windungen glatt auslaufen, zu Ösen gebogen, in Form eines Schraubenloches vorhanden oder durch einen Quersteg mit der Nachbarwindung verbunden sind.

6. Osteosyntheseplatte gemäß einem der Ansprüche 1 bis 5, welche zumindest ein Quersteg aufweist um zwei Windungen miteinander zu verbinden, wobei vorzugsweise der Quersteg ein integriertes Schraubenloch aufweist.

7. Osteosyntheseplatte gemäß einem der Ansprüche 1 bis 6, **dadurch gekennzeichnet, daß** die Querschnittsfläche des Materials der Windung entweder überall gleich ist oder abschnittsweise unterschiedlich, wobei die Form des Querschnittes rund, eckig, eine Kombination daraus, oder eine der drei Varianten kombiniert mit geraden Abschnitten ist.

8. Osteosynthesesystem mit mehreren Osteosyntheseplatten gemäß einem der Ansprüche 1 bis 7.

9. Apparat zur Distraktionsosteogenese, **dadurch gekennzeichnet, daß** er eine Osteosyntheseplatte gemäß einem der Ansprüche 1 bis 7 als Bestandteil enthält.

10. Osteosyntheseplatte gemäß einem der Ansprüche 1 bis 8, **dadurch gekennzeichnet, dass** die Osteosyntheseplatte aus einem resorbierbaren Material ausgebildet ist.

11. Osteosyntheseplatte gemäß einem der Ansprüche 1 bis 8, **dadurch gekennzeichnet, dass** die Osteosyntheseplatte aus einem nicht resorbierbaren Material ausgebildet ist.

12. Osteosynthesesystem mit einer Osteosyntheseplatte gemäß einem der Ansprüche 1 bis 11 und einer Beilagscheibe, **dadurch gekennzeichnet, dass** die Beilagscheibe ein zentrales Schraubenloch und 3 Domen, welche ein gleichseitiges Dreieck aufspannen, besitzt.

## Claims

1. Osteosynthesis plate which can be pressed against the surface of the bone and fixed with screws, preferably screws and a shim washer, whereby the osteosynthesis plate is constructed in the shape of a spiral,
**characterised by** the fact that
the spiral starts from a central point and extends in curved or angular coils which run in parallel to one another peripherally,
whereby the section of the spiral's coil fixed by the screw is pressed against the surface of the bone via the screw head or shim washer, where it is held in place.

2. Osteosynthesis plate in accordance with requirement 1, **characterised by** the fact that the coils contain curved and angular sections.

3. Osteosynthesis plate in accordance with requirement 1 or 2, **characterised by** the fact that individual sections of the spiral are not curved, but straight, particularly the spirals in the shape of a horseshoe, a T-shape and a triangle.

4. Osteosynthesis plate in accordance with one of the requirements between 1 and 3, **characterised by** the fact that the spiral contains at least one fixation element in the form of a screw hole, whereby the screw hole's edge is preferably circular, angular or a combination of the two, or one of the three variations combined with straight sections.

5. Osteosynthesis plate in accordance with requirement 4, **characterised by** the fact that the coil ends terminate smoothly, are bent into loops in the shape of a screw hole or are connected to the neighbouring coil via a transverse bar.

6. Osteosynthesis plate in accordance with one of the requirements between 1 and 5, which features at least one transverse bar in order to connect two coils, whereby the transverse bar preferably features an integrated screw hole.

7. Osteosynthesis plate in accordance with one of the requirements between 1 and 6, **characterised by** the fact that the coil material's cross-sectional surface is either completely identical or contrasts in parts, whereby the cross-section's shape may be circular, angular or a combination of the two, or one of the three variations combined with straight sections.

8. Osteosynthesis system with several osteosynthesis plates in accordance with one of the requirements between 1 and 7.

9. Apparatus used for distraction osteogenesis, **characterised by** the fact that it contains an osteosynthesis plate as a component in accordance with one of the requirements between 1 and 7.

10. Osteosynthesis plate in accordance with one of the requirements between 1 and 8, **characterised by** the fact that the osteosynthesis plate is made from a resorbable material.

11. Osteosynthesis plate in accordance with one of the requirements between 1 and 8, **characterised by** the fact that the osteosynthesis plate is made from a non-resorbable material.

12. Osteosynthesis system with an osteosynthesis plate in accordance with one of the requirements between 1 and 11 and a shim washer, **characterised by** the fact that the shim washer features a central screw hole and 3 bolts, which span an equilateral triangle.

## Revendications

1. La plaque d'ostéosynthèse qui peut être fixée au moyen de vis, de préférence au moyen de vis et d'une rondelle de serrage, en la serrant contre la surface de l'os, auquel cas la plaque d'ostéosynthèse est constituée sous forme de spirale, et est
**caractérisée par le fait que**
la spirale démarre à partir d'un point central et se prolonge en spire courbées ou anguleuses qui passent chacune parallèlement les unes aux autres et se dirigent vers la périphérie ;
pour cela, le segment de la spire de la spirale qui est fixé par la vis est serré par la tête de vis ou par la rondelle de serrage sur la surface de l'os et y est maintenue en place.

2. La plaque d'ostéosynthèse conformément à la revendication 1 est **caractérisée par le fait que** les spires comportent des segments recourbés et des segments anguleux.

3. La plaque d'ostéosynthèse conformément à l'une des revendications 1 à 2 est **caractérisée par le fait que** certains segments de la spirale ne sont pas recourbés mais droits, en particulier.que la spirale présente une forme en fer à cheval, une forme en T ou une forme triangulaire.

4. La plaque d'ostéosynthèse conformément à l'une des revendications 1 à 3 est **caractérisée par le fait que** la spirale comporte au moins un élément d'ancrage sous forme d'un trou destiné à une vis, auquel cas la délimitation du trou destiné à la vis est de préférence rond, anguleux ou une combinaison des deux, ou une des trois variantes combinée avec des segments droits.

5. La plaque d'ostéosynthèse conformément à la revendication 4 est **caractérisée par le fait que** les extrémités des spires se terminent de manière lisse, sont recourbées pour former une boucle, existent sous forme d'un trou destiné à une vis ou sont reliées par une entretoise avec la spire voisine.

6. La plaque d'ostéosynthèse conformément à l'une des revendications 1 à 5 dispose au moins d'une entretoise pour relier ensemble deux spires, et, dans ce cas, l'entretoise dispose de préférence d'un trou intégré destiné à une vis.

7. La plaque d'ostéosynthèse conformément à l'une des revendications 1 à 6 est **caractérisée par le fait que** la surface de la section du matériel de la spire est soit partout pareille, soit que des segments sont différents, auquel cas, la forme de la section est arrondie, anguleuse, une combinaison des deux ou une des trois variantes combinée avec des segments droits.

8. Le système d'ostéosynthèse avec plusieurs plaques d'ostéosynthèse conformément à l'une des revendications 1 à 7.

9. Appareil destiné à la distraction osseuse, **caractérisé par le fait qu'**il comprend en tant que partie intégrante une plaque d'ostéosynthèse conformément à l'une des revendications 1 à 7.

10. La plaque d'ostéosynthèse conformément à l'une des revendications 1 à 8 est **caractérisée par le fait que** la plaque d'ostéosynthèse est formée de matériel résorbable.

11. La plaque d'ostéosynthèse conformément à l'une des revendications 1 à 8 est **caractérisée par le fait que** la plaque d'ostéosynthèse est formée de matériel non résorbable.

12. Le système d'ostéosynthèse avec une plaque d'ostéosynthèse conformément à l'une des revendications 1 à 11, et une rondelle de serrage, est **caractérisé par le fait que** la plaque de serrage possède un trou central destiné à une vis et 3 dômes qui se tendent pour former un triangle à côtés égaux.
